Europäisches Patentamt

European Patent Office

Office européen des brevets

(11) Publication number:

**0 341 733**
**A2**

(12) **EUROPEAN PATENT APPLICATION**

(21) Application number: **89108591.2**

(22) Date of filing: **12.05.89**

(51) Int. Cl.⁴: **C07K 3/20 , C07K 13/00 , //A61K39/29**

(30) Priority: **13.05.88 KR 556488**

(43) Date of publication of application:
**15.11.89 Bulletin 89/46**

(84) Designated Contracting States:
**AT BE CH DE ES FR GB IT LI NL SE**

(71) Applicant: **DONG-A PHARM. CO., LTD.**
**252, Yongdoo-dong,**
**Dongdaemoon-ku Seoul(KR)**

(72) Inventor: **Won Bae, Kim**
**102-503, Hyundae Apt. House,Kaepo-dong**
**Kangnam-ku, Seoul(KR)**
Inventor: **Byung Moon, Kim**
**50-28, Myunmok-6-dong**
**Jungryang-ku, Seoul(KR)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Isolation of HBsAg by immunoaffinity chromatography using the anti-idiotype antibody as an eluting agent.**

(57) A immunoaffinity method for purification of Hepatitis B surface antigen (HBsAg) using monoclonal anti-idiotype (anti-Id) as a specific eluent, is described. Ammonium sulfate fractions of Hepatitis B surface antigen (HBsAg) containing plasma were adsorbed on monoclonal idiotype (Id) immunoadsorbents. Bound material was eluted from the column with the anti-Id of HBsAg. The eluate, contaminating anti-Id, was further purified by gel filtration. The procedure yielded highly purified HBsAg. The final HBsAg Product was free from contaminating humen plasma and mouse ascite proteins. The procedure is generally applicable and particularly attractive when the antigen is unstable in commonly used eluting conditions.

**Isolation of HBsAg by Immunoaffinity Chromatography using the anti-idiotype antibody as an eluting agent**

1. Field of invention

The present invention relates to a method for the isolation of HBsAg by immunoaffinity chromatography using the anti-idiotype antibody as an eluting agent

2. Description of prior art

Affinity chromatography is widely used for the isolation of biologically active compounds, and particularly immunoaffinity techniques are widely used as a selective isolation method using the specific affinity and the selectivity of the antigen-antibody complex. Immunoaffinity chromatography is an isolation procedure which comprises preparing specific antibodies against biological compounds (antigen), immobilizing the above antibody (ligand) by covalently bonding it to a solid matrix, absorbing selectively specific antigenic compounds to the immobilized antibody by allowing complex sample solutions to come in contact and react with the immobilized antibody, desorbing and eluting the bound specific compounds from the immobilized ligand by using an eluting agent. These techniques provide a simple, rapid and quantitative procedure for isolating biologically active compounds, compared with the isolating methods such as gel filtration and ion exchange chromatography. The successful application of these techniques generally depends on the finding of eluting conditions that the biological activity of the antigenic compounds will be withstood in the elution step. Various eluting conditions have been used for the immunoaffinity techniques. The generally used eluting conditions including pH change, denaturants, chaotropic ions, polarity reducing and ionic strength change are severe and case denaturation and loss of the biological activity of the eluted compounds.

As a result, the activity yield and purity of purified compounds decrease in the elution step. However, these problems may be circumvented by using specific eluting agents that can elute the compounds from immunoadsorbents in physiological conditions such as the affinity purification of certain enzymes using inhibitors or cofactors as specific eluting agents in mild conditions.

These specific affinity elution techniques have not been utilized for immunoaffinity purification of the biologically active compounds, because these eluting agents which bind to the ligand specifically have not been found. Recently there are antibodies of anti-idiotypes that have been designated as internal images of antigenic proteins, and they can function operationally as effective surrogate antigens. Therefore, we have investigated the value of antibodies of anti-idiotypes as specific eluents for the purification of biologically active compounds, using the Hepatitis B surface antigen as a model system.

3. Detailed description of the invention

The present invention presents the isolation method of Hepatitis B surface antigen (HBsAg) from the blood of viral hepatitis B carrier by immunoaffinity techniques involving the use antibodies of anti-idiotypes as eluting agents. This invention provides the procedures for simple and rapid recovery of highly purified HBsAg with a minimum loss of activity occurring in the elution step.

Also these techniques can be applied in the isolation of biologically active compounds which are difficult to isolate by other techniques. Particularly these techniques can be used as a economic method to isolate the biologically active compounds which are easily denatured under the severe eluting conditions. The image bearing anti-idiotype antibody of an antigen was used as an eluting agent in this invention. This antibody of anti-idiotype competes with the antigen molecules against the ligand antibody, and replaces the bound antigen molecules to the ligand antibody. Therefore, the anti-idiotype of antibody is able to elute the antigen molecules from the immunosorbents in the isolation of the biologically active compounds(antigen) by the immunoaffinity chromatography. Monoclonal or polyclonal anti-idiotype antibodies which are used as a eluting agent can be easily prepared by the previously established method(Thanavala et al., 1985). Since this invention adapts the physicochemically stable conditions instead of the severe conditions in the elution step, the biologically active compounds can be isolated with high purity and activity yield without eluting the contaminating material which is nonspecifically absorbed to the ligand. The antibodies of anti-idiotype contaminated in elution step, can be easily separated and removed by gel filtration. Therefore, this invention may be widely applied in the isolation of biologically active compounds such as followings.

1) The purified isolation of biologically active proteins, eg., insulin, urokinase, α-IFN, β-IFN, γ-IFN, EPO, secretin, TNF, CSF, HCG, TPA, Factor VIII or HBsAg from secretions or body fluids of human or animal such as blood, abdominal dropsy, intestinal fluids, ascitic fluids, urine and semen.

2) The purified isolation of the biologically active proteins, e.g., insulin, HCG, $\alpha$-IFN, $\beta$-IFN, $\gamma$-IFN, TNF, HBsAg, IL-1,2,3, 4, secretin, somatostatin or urokinase from the culture supernatants of normal cells or transformed cell lines obtained from human or animal, and the culture supernatants of genetically engineered cells obtained by recombinant DNA techniques or cell fusion.

3) In case of purification of biologically active compounds from 2) as a before treatment, we can be smashed the cell to pieces by published methods, centrifusing, salting-out, dialysis, concentration etc.

4) Purification of extracted biologically active compounds such as protein, lipid, lipoprotein, nucleotide, nucleoprotein, polysaccharide from body fluids of human or animals or cell-culture solution or cultured cell line, we can applied method from 1) to 3).

5) Purification of biologically active compounds from 1) to 4) especially antigen or indicated materials such as HBsAg, CALLA, $\beta$-HCG, $\gamma$-Fetoprotein and conjugated materials with radio isotope, biotin, avidin, HRPO, alkaline phosphatases, using this method result to get high-yield ratio, high degree purity.

The present invention shall be described in details as follows :

1. Preparation of a image bearing antibody of anti-idiotype HBsAg.

Mouse monoclonal idiotypes and monoclonal anti-idiotype specific for a group specific a determinant of HBsAg in this invention are produced and characterized by previously established methods (Thanavala et al., 1985). A monoclonal anti-idiotype with high affinity against monoclonal anti-HBsAg antibodies is selected and used in this invention. The monoclonal antibodies are purifed from mouse ascitic fluids or culture supernatants of mouse hybridoma cells by hydroxylapatite chromatography.

2. Preparation of immunoadsorbents

Immunoadsorbents are prepared by the common procedures using monoclonal antibodies as a ligand. That is, purified monoclonal idiotypes specific for HBsAg are covalently coupled to a solid matrix to a appropriate ratio of ligand antibody per ml of matrix gels for ligand immobilization. The coupled gels are blocked to remove any remaining active group s, and equilibrated with a neutral buffer, e.g. phosphate-buffered saline(0.01M, pH 7.2).

3. Immunoaffinity chromatography

The immunoadsorbents are packed into a glass column, and equilibrated with a neutral buffer. HBsAg containing solutions, which are obtained from HBsAg positive sera by the previously established method (Peterson, 1981), e.g., salting out, are absorbed on the column, and HBsAg molecules are bound to the ligand antibodies on the immunoadsorbents. Also, after HBsAg containing solutions are absorbed directly to the immunoadsorbents and incubated by rotating end-over-end, the immunoadsorbents can be packed into a column. The column is then extensively washed with a washing solution, e.g., phosphate-buffered saline (pH 7.2) until negligible amounts of non-specifically bound materials are eluted. The bound HBsAg are eluted by adding and recycling the anti-idiotype solution as a eluting agent for different times at different temperature, anti-idiotypes are dissolved and used in a neutral buffer, e.g., phosphate-buffered saline (0.01M, pH 7.2). Eluted fractions containing proteins, as determined by optical density of the fractions at 280nm, are pooled and concentrated by ultrafiltration.

4. Isolation of HBsAg by gel filtration

HBsAg molecules are seperated from anti-idiotype contaminants of HBsAg containing fractions by gel filtration. HBsAg containing fractions concentrated after eluting from affinity column are applied to a column packed with gel filtration media, e.g., Sepharose 4B(pharmacia) or Ultrogel (LKB) and eluted with a neutral buffer containing salts. The fractions are collected and the optical density reads at 280nm. The HBsAg containing fractions are pooled, dialysed against distilled water, and lyophilized.

Example 1

HBsAg was isolated from 14 litre of HBsAg containing sera as follows.

Crude HBsAg mixtures in 355ml of phosphate-buffered saline (0.01M, pH 7.2) were prepared from 14 litre of sera by the previously established method (Peterson, 1981) and used as a starting material. Total protein contents of the crude HBsAg mixtures were 12.1g and the specific activity of HBsAg was measured as 1:4000 per 1mg protein.

HBsAg was determined by reverse passive haemagglutination assay(RPHA), and expressed as dilution titer per mg protein.

## 1) Immunoaffinity chromatography

453mg of purified mouse monoclonal anti-HBsAg 1gG was coupled to 25g of CNBr activated Sepharose 4B(Pharmacia Fine Chemicals, Sweden) by the common procedure, and then 87ml of the Sepharose gel immobilized with monoclonal idiotypes(immunoadsorbents) was packed into a glass column (5 X 30cm). The column was equilibrated with phosphate-buffered saline(0.01M, pH 7.2). To the column, 355ml of the crude HBsAg mixtures was added and incubated by circulating the mixture for 20 hours at 4°C at a speed of 1.5ml per minute. The column was then washed with 1 litre of phosphate-buffered saline (0.01M, pH 7.2). 500mg of purified mouse monoclonal anti-idiotypes of HBsAg (Thanavala et al., 1985) was dissolved in 100ml of phosphate-buffered saline (0.01M, pH 7.2), and used as an eluting agent. The bound HBsAg was eluted by adding and recycling 100ml of anti-idiotype eluants (5mg/ml) to the column for 18 hours at 4°C or 4 hours at room temperature at a speed of 1ml per minute. Eluted fractions containing protein were collected and concentrated to 25ml by ultrafiltration using a PM30 membrane (Amicon Wright, Amersham).

## 2) Gel filtration

HBsAg was seperated from anti-idiotype contaminants of eluted fractions containing proteins by gel filtration. 1 litre of Sepharose 48 gel (Pharmacia Fine Chemical, Sweden) was packed into a glass column (4X 85cm), and equllibrated with phosphate-buffered saline (0.01M, pH 7.2). 25ml of HBsAg containing fractions concentrated after eluting from affinity column were applied to a Sepharose 48 column equilibrated with phosphate-buffered saline(0.01M, pH 7.2), and then 5ml fractions were collected with phosphate-buffer saline-(0.01M, pH 7.2). HBsAg containing fractions were pooled, dialysed against distilled water, and lyophilized. Total protein contents of purified HBsAg preparation were 217mg and the specific activity was measured as 1:200,000 per mg by RPHA. There was 50 fold increase in the specific activity of HBsAg yield 74. 7% of the original actinity, in purified HBsAg preparation on these isolation procedure. Also, in purified HBsAg preparation, the other serum proteins were not detected by SDS-PAGE or immunoelectrophoresis(Figure 1).

## Example 2

HBsAg was isolated from 14 litre of HBsAg containing sera according to Example 1 except using a batch type in a sample application of immunoaffinity step. That is, 355ml of the crude HBsAg mixture was directly added to the 87ml of Sepharose gel immobilized with idiotypes and incubated by, rotating end-over-end for 20 hours at 4°C. After removing the supernatant of the gel, the immunoadsorbents absorbed with HBsAg were packed into a glass column (5x 30cm). Finally, total protein contents of purified HBsAg were 220mg and the specific activity was measured as 1:200,000. There was 50 fold increse in the specific activity of HBsAg, yielding 75.2% of the original activity, in purified HBsAg preparation.

## Claims

1. A method for isolating HBsAg by immunoaffinity chromatography using the anti-idiotype antibody of HBsAg as an eluting agent.

2. The method according to claim 1 wherein the starting materials are selected from blood or body fluid of mammals or culture supernatants of genetically engeneered mammalian cells.

3. The method according to claim 1 wherein the elution is carried out in physicochemical conditions.

4. The method according to claim 3 wherein the elution is carried out at temperatures of about 4°C to 5°C.

5. The method according to claim 3 wherein the elution is carried out at a pH of about 5 to 9.

6. The method according to claim 3 wherein the elution is carried out by a period of about 30 minutes to 1,200 minutes.

7. The method according to claim 3 wherein the amount of the anti-idiotype in the eluting solution is present in about 0.1 to 1000 per 100 parts of HBsAg by weight.